Europäisches Patentamt

European Patent Office

Office européen des brevets

(19)

(11) **EP 1 286 648 B1**

(12) **FASCICULE DE BREVET EUROPEEN**

(45) Date de publication et mention
de la délivrance du brevet:
**05.10.2005 Bulletin 2005/40**

(21) Numéro de dépôt: **01943571.8**

(22) Date de dépôt: **06.06.2001**

(51) Int Cl.⁷: **A61K 7/16**, C01B 33/193

(86) Numéro de dépôt international:
**PCT/FR2001/001742**

(87) Numéro de publication internationale:
**WO 2001/093803 (13.12.2001 Gazette 2001/50)**

(54) **UTILISATION D'UNE SILICE DE PRECIPITATION DE HAUTE STRUCTURE ET DISPERSIBLE COMME AGENT EPAISSISSANT OU TEXTURANT DANS LES COMPOSITIONS DENTIFRICES**

VERWENDUNG VON DISPERGIERBARER DICHTER FÄLLUNGSKIESELSÄURE MIT HOHER STRUKTUR ALS VERDICKUNGSMITTEL UND TEXTURSMITTEL IN ZAHNPFLEGEMITTELN

USE OF A HIGH STRUCTURE AND DISPERSIBLE PRECIPITATED SILICA AS THICKENING OR TEXTURING AGENT IN TOOTHPASTE COMPOSITIONS

(84) Etats contractants désignés:
**AT BE CH CY DE DK ES FI FR GB GR IE IT LI LU MC NL PT SE TR**

(30) Priorité: **07.06.2000 FR 0007288**

(43) Date de publication de la demande:
**05.03.2003 Bulletin 2003/10**

(73) Titulaire: **RHODIA CHIMIE**
**92512 Boulogne Billancourt Cedex (FR)**

(72) Inventeurs:
 • **CHEVALLIER, Yvonick**
  **F-69270 FONTAINES SAINT MARTIN (FR)**
 • **DROMARD, Adrien**
  **F-69006 LYON (FR)**
 • **LAHARY, Pierre-Yves**
  **F-69006 LYON (FR)**
 • **MARCANDELLI, Céline**
  **F-75010 PARIS (FR)**

(74) Mandataire: **Fabre, Madeleine-France et al Rhodia Services,**
**Direction de la Propriété Industrielle,**
**40, rue de la Haie-Coq**
**93306 Aubervilliers Cedex (FR)**

(56) Documents cités:
 **EP-A- 0 345 109**       **WO-A-00/64810**
 **WO-A-97/46485**         **WO-A-99/36360**
 **US-A- 5 419 888**       **US-A- 5 484 581**
 **US-A- 5 647 903**

 • **PATENT ABSTRACTS OF JAPAN vol. 009, no. 108 (C-280), 11 mai 1985 (1985-05-11) & JP 60 001115 A (CENTRAL GLASS KK), 7 janvier 1985 (1985-01-07)**
 • **PATENT ABSTRACTS OF JAPAN vol. 011, no. 297 (C-448), 25 septembre 1987 (1987-09-25) & JP 62 087507 A (FUJI DEBUISON KAGAKU KK), 22 avril 1987 (1987-04-22)**

Remarques:
Le dossier contient des informations techniques présentées postérieurement au dépôt de la demande et ne figurant pas dans le présent fascicule.

EP 1 286 648 B1

## Description

La présente invention a pour objet l'utilisation, dans les compositions dentifrices, d'une silice de précipitation de haute structure, hautement dispersible et de préférence dense, comme agent épaississant ou texturant ; elle a également pour objet un procédé pour épaissir les compositions dentifrices ou apporter de la texture aux compositions dentifrices, par incorporation auxdites compositions d'une silice de précipitation de haute structure, hautement dispersible et de préférence dense, ainsi que les compositions dentifrices ainsi obtenues.

Il est connu d'utiliser des silices, notamment des silices de précipitation de haute structure (prise d'huile DOP d'au moins 200 ml/g), de fine granulométrie (généralement de diamètre médian inférieur à 15 µm) et de faible densité comme agent épaississant dans les compositions dentifrices.

Le document US-A-5 484 581 décrit des silices de précipitation destinées à cet usage.

La Demanderesse a trouvé que des silices de précipitation de haute structure de diamètre médian d'au moins 20µm, hautement dispersibles et de préférence denses, présentent un pouvoir épaississant tout à fait remarquable dans les compositions dentifrices.

Un premier objet de l'invention consiste en l'utilisation, en tant qu'agent épaississant ou texturant dans les compositions dentifrices, d'une silice de précipitation présentant

.    un pH de 3,5 à 9 , de préférence de 4 à 9, tout particulièrement de 5 à 8
.    une prise d'huile DOP supérieure à 200ml/g, de préférence supérieure à 230ml/g, tout particulièrement supérieure à 250ml/g
.    une surface spécifique CTAB de 70 à 250 $m^2/g$, de préférence de 100 à 200 $m^2/g$
.    un diamètre médian d'au moins 20µm, de préférence d'au moins 25µm déterminé par diffraction laser sans ultrasons, jusqu'à 600µm
.    un taux d'anion résiduel, exprimé en sulfate de sodium, inférieur à 5% en poids, de préférence inférieur à 3 % en poids.

Le pH de la silice est mesuré selon la norme ISO 787/9 (pH d'une suspension à 5% en poids de silice dans l'eau désionisée).

La surface spécifique CTAB est la surface externe déterminée selon la norme NFT 45-007 (novembre 1987).

Les silices selon l'invention présentent de préférence une surface spécifique BET telle que le rapport surface spécifique BET / surface spécifique CTAB est de 0,9 à 1,7 , de préférence de 0,9 à 1,5 , tout particulièrement de 0,9 à 1,4 .

La surface spécifique BET est déterminée selon la méthode de BRUNAUER - EMET - TELLER décrite dans "The journal of the American Chemical Society", Vol. 60, page 309, février 1938 et correspondant à la norme NFT 45007 (novembre 1987).

La prise d'huile DOP est déterminée selon la norme ISO 787/5 en mettant en oeuvre du dioctylphtalate.

Les silices selon l'invention présentent de préférence une densité de remplissage à l'état tassée d'au moins 0,17 g/ml, tout préférentiellement d'au moins 0,18 g/ml, et encore plus particulièrement d'au moins 0,19 g/ml, voire même d'au moins 0,20 g/ml ; la densité de remplissage à l'état non-tassée est de préférence d'au moins 0,13 g/ml, tout préférentiellement d'au moins 0,15 g/ml, et encore plus particulièrement d'au moins 0,16 g/ml.

La densité de remplissage à l'état tassée ou non-tassée est déterminée selon la norme ISO 787/11.

Le diamètre médian est déterminé par diffraction laser selon la norme NF X 11-666. Le granulomètre utilisé peut être par exemple du type SYMPATEC ou MALVERN.

On donne ci-après des exemples de ce type de granulomètre avec les critères de mesures utilisés :

Granulomètre SYMPATEC HELOS

*    dispersion en voie humide : SUCELL

Critères de mesure

*    concentration optique : 20%
*    liquide de mesure : eau déminéralisée dégazée
*    absence d'ultrasons

* absence de dispersant
* focale : 100 mm
* durée de la mesure : 20 secondes

Granulomètre MALVERN MASTERSIZER MICROPLUS

Critères de mesure

* concentration optique : 12% ± 2
* liquide de mesure : eau déminéralisée dégazée
* absence d'ultrasons
* absence de dispersant
* durée de la mesure : 10 secondes

Pour une bonne réalisation de l'invention, ladite silice présente un facteur de désagglomération aux ultra-sons $F_D$ d'au moins 8, de préférence d'au moins 9,5 , avec mesure granulométrique par diffraction laser à l'aide d'un granulomètre SYMPATEC.

Le facteur de désagglomération $F_D$ est déterminé selon le protocole suivant : l'aptitude à la désagglomération ou à la dispersion des particules de silice est appréciée par une mesure granulométrique (par diffraction laser), effectuée sur une suspension de silice préalablement désagglomérée par ultra-sonification ; on mesure ainsi l'aptitude à la désagglomération de la silice (rupture des objets de 0,1 à quelques dizaines de microns). La désagglomération sous ultra-sons est effectuée à l'aide d'un sonificateur VIBRACELL BIOBLOCK (600 W), équipé d'une sonde de diamètre 19 mm. La mesure granulométrique est effectuée par diffraction laser sur un granulomètre SYMPATEC.

On pèse dans un pilulier (hauteur : 6 cm et diamètre : 4 cm) 2 grammes de silice et l'on complète à 50 grammes par ajout d'eau permutée : on réalise ainsi une suspension aqueuse à 4 % de silice qui est homogénéisée pendant 2 minutes par agitation magnétique. On procède ensuite à la désagglomération sous ultra-sons comme suit : la sonde étant immergée sur une longueur de 4 cm, on règle la puissance de sortie de manière à obtenir une déviation de l'aiguille du cadran de puissance indiquant 20 %. La désagglomération est effectuée pendant 420 secondes. On réalise ensuite la mesure granulométrique en introduisant dans la cuve du granulomètre un volume V (exprimé en ml) de la suspension homogénéisée nécessaire pour obtenir une concentration optique de 20%.
Le facteur de désagglomération $F_D$ est alors donné par l'équation :

$$F_D = 10 \times V / \text{concentration optique de la suspension mesurée par le granulomètre}$$

(cette concentration optique est de l'ordre de 20%)
Ce facteur de désagglomération $F_D$ est indicatif du taux de particules de taille inférieure à 0,1µm qui ne sont pas détectées par le granulomètre. Ce facteur est d'autant plus élevé que la silice présente une aptitude à la désagglomération élevée.

La valeur du diamètre médian $\emptyset_{50}$ que l'on obtient selon ce test est d'autant plus faible que la silice présente une aptitude à la désagglomération élevée.

L'aptitude de la silice, selon l'invention, à se disperser dans une formulation dentifrice peut également être déterminée par mesure du diamètre médian d50 de la silice au granulomètre MALVERN MASTERSIZER après désagglomération par ultra-sons selon le test de dispersion suivant :
La puissance des ultra-sons dans le granulomètre MALVERN MASTERSIZER étant réglée sur la graduation maximale de 20, on introduit une quantité de silice de façon à obtenir une concentration optique de 12 ± 2%.
On mesure le diamètre médian d50 et le pourcentage de particules de silice de diamètre supérieur à 51µm après avoir maintenu les ultra-sons dans la cuve pendant 60 secondes, la cuve étant homogénéisée par circulation de la suspension au moyen d'une pompe centrifuge. La mesure est enregistrée 10 secondes après l'arrêt des ultra-sons.
Pour la bonne réalisation de l'invention, le diamètre médian est d'au plus 40µm, de préférence d'au plus 35µm. Le pourcentage en poids de particules de silice de diamètre supérieur à 51 µm est favorablement d'au plus 30, de préférence d'au plus 25.

Ladite silice de haute structure, hautement dispersible et de préférence dense, peut être obtenue par réaction d'une solution aqueuse de silicate de métal alcalin avec un agent acidifiant pour former une bouillie de silice, puis séparation, acidification éventuelle et séchage du gâteau de silice,

\*   ladite bouillie étant formée à une température de 60 à 98°C, par réaction d'une solution aqueuse de silicate de métal alcalin et d'un agent acidifiant selon les étapes suivantes (opération de formation de bouillie) :

(a) une première étape, consistant à mettre en oeuvre un pied de cuve initial comportant de l'eau et tout ou partie du silicate de métal alcalin, selon une concentration, exprimée en silice, inférieure ou égale à 100 g/l, de préférence inférieure ou égale à 80 g/l ;

(b) une deuxième étape consistant à introduire en continu ou discontinu un agent acidifiant, jusqu'à obtenir un pH du milieu d'au moins 7, de préférence de 7 à 9,2 ;

(c) le cas échéant, une troisième étape, consistant à introduire simultanément la quantité restante de silicate et un agent acidifiant, en maintenant la température du milieu réactionnel constante et un pH d'au moins 7, de préférence de 7 à 9,2 ;

(d) et une étape finale d'acidification du milieu réactionnel par addition d'un agent d'acidification jusqu'à obtenir un pH de la bouillie de 3 à 6, de préférence de 4 à 6 ;

\*   et étant ensuite séparée par filtration/lavage et fluidifiée jusqu'à obtenir un gâteau de silice de perte au feu supérieure à 80%, de préférence d'au moins 82% et un taux d'anion résiduel, exprimé en sulfate de sodium, inférieur à 5% en poids, de préférence inférieur à 3 % en poids, par rapport au poids deproduit final.

Le choix de l'agent acidifiant et du silicate pour la réalisation de l'opération de formation de bouillie se fait d'une manière bien connue en soi.

On peut rappeler que l'on utilise généralement comme agent acidifiant un acide minéral fort tel que l'acide sulfurique, l'acide nitrique ou l'acide chlorhydrique, ou un acide organique tel que l'acide acétique, l'acide formique ou l'acide carbonique.

L'agent acidifiant peut être mis en oeuvre sous forme dilué ou concentré.

En particulier, lorsqu'il s'agit d'acide sulfurique, il peut être mis en oeuvre sous forme d'une solution aqueuse contenant de 40 à 400 g/l, de préférence de 60 à 150g/l d'acide.

On peut utiliser en tant que silicate toute forme courante de silicates en solution aqueuse, tels que métasilicates, disilicates et avantageusement un silicate de métal alcalin, notamment un silicate de sodium ou de potassium.

La solution de silicate peut présenter une concentration exprimée en silice comprise entre 20 et 350 g/l, par exemple entre 60 et 300 g/l, en particulier entre 100 et 260 g/l.

Dans le cas où l'on utilise un silicate de sodium, celui-ci présente, en général, un rapport pondéral $SiO_2/Na_2O$ compris entre 2 et 4, par exemple entre 3,0 et 3,7.

De manière plus particulière, on emploie, comme agent acidifiant, l'acide sulfurique, et, comme silicate, un silicate de sodium.

La première étape de l'opération de formation de bouillie (étape (a)) consiste à mettre en oeuvre un pied de cuve qui comprend de l'eau et du silicate.

D'une manière préférentielle, la quantité de silicate (exprimée en $SiO_2$) présente dans le pied de cuve initial ne représente qu'une partie de la quantité totale de silicate (exprimée en $SiO_2$) engagée dans la réaction.

Cette quantité partielle de silicate (exprimée en $SiO_2$) peut représenter par exemple jusqu'à 95% de la quantité totale de silicate ; cette quantité est de préférence d'au moins 5% de la quantité totale de silicate (exprimée en $SiO_2$).

La concentration en silicate dans le pied de cuve initial est inférieure ou égale à 100 g de $SiO_2$ par litre. De préférence, cette concentration est inférieure ou égale à 80g/l. Celle-ci est de préférence d'au moins 5g/l.

La deuxième étape (étape (b)) consiste à ajouter l'agent acidifiant dans le pied de cuve.

Cette addition qui entraîne une baisse corrélative du pH du milieu réactionnel se fait jusqu'à ce qu'on atteigne une valeur du pH de préférence de 7 à 9,2.

La troisième étape (étape (c)) est réalisée si le pied de cuve de départ ne comprend qu'une partie de la quantité totale de silicate engagé dans la réaction.

L'addition simultanée d'agent acidifiant et de la quantité restante de silicate est de préférence réalisée de manière telle que la valeur du pH soit constamment égale (à ± 0,2 près) à celle atteinte à l'issue de l'étape (b).

Cette étape est réalisée à une température constante correspondant de préférence à celle de la fin de l'étape (b).

A l'étape (d) d'acidification finale, on ajoute au milieu réactionnel issu de l'étape (c) si une partie seulement de la quantité totale de silice a été mise en oeuvre dans le pied de cuve, ou de l'étape (b) si la quantité totale de silice a été mise en oeuvre dans le pied de cuve, une quantité supplémentaire d'agent acidifiant, et ce jusqu'à l'obtention d'une valeur du pH du milieu réactionnel comprise entre 3 et 6, de préférence entre 4 et 6.

Il peut être avantageux d'effectuer une ou plusieurs étapes de mûrissement intermédiaires lors de l'opération de formation de bouillie.

Ainsi les étapes (b) et/ou (c) et/ou (d) peuvent être suivies d'une étape de mûrissement.

Il est notamment avantageux d'effectuer après l'étape d'acidification finale (d), un mûrissement du milieu réactionnel, ce mûrissement pouvant par exemple durer de 1 à 30 minutes, notamment de 2 à 15 minutes.

Il peut être également bénéfique d'effectuer, après l'étape (b) de première addition d'acide, un mûrissement du milieu réactionnel.

La température du milieu réactionnel pendant l'opération de formation de bouillie est généralement comprise entre 60 et 98 °C. On peut conserver la même température pendant toute la réaction ou adopter un profil de température non uniforme.

Selon un premier mode de réalisation, l'opération de formation de bouillie est effectuée à une température constante, de préférence comprise entre 75 et 98°C.

Selon un deuxième mode de réalisation (préféré), la température de fin de réaction est plus élevée que la température de début de réaction ; ainsi, on maintient la température au début de l'opération de préférence entre 60 et 80 °C, puis on augmente la température, pour atteindre de préférence une valeur comprise entre 75 et 98 °C, notamment à la fin de l'étape (b) de première addition d'acide, valeur à laquelle la température est maintenue jusqu'à la fin de la réaction.

Une variante de réalisation de l'opération de formation de bouillie, consiste à réaliser au moins une des étapes (a) à (c) ci-dessus en présence d'un agent électrolyte.

Le terme d'électrolyte s'entend ici dans son acceptation normale, c'est-à-dire qu'il signifie toute substance ionique ou moléculaire qui, lorsqu'elle est en solution dans l'eau, se décompose ou se dissocie pour former des ions ou des particules chargées.

Parmi les électrolytes on peut citer notamment les sels de métaux alcalins ou alcalino-terreux, notamment le sel du métal du silicate de départ et de l'agent acidifiant, à savoir de préférence le sulfate de sodium ; les chlorure, nitrate et hydrogénocarbonate de sodium sont également intéressants.

Celui-ci est mis en oeuvre à au moins une des étapes (a) à (c) selon une quantité pouvant être de l'ordre de 0,05 à 0,3 mole/litre lorsqu'il s'agit d'un sel électrolyte de métal alcalin ou de l'ordre de 0,005 à 0,05 mole/litre lorsqu'il s'agit d'un sel électrolyte de métal alcalino-terreux.

D'une manière préférentielle, l'agent électrolyte, est mis en oeuvre dans le pied de cuve (étape (a)).

Selon cette variante de réalisation (mise en oeuvre d'un agent électrolyte à au moins une des étapes (a) à (c)), la concentration en silicate de métal alcalin, exprimée en silice, dans ledit pied de cuve à l'étape (a) est de préférence supérieure ou égale à 40g/l ; le pH du milieu réactionnel aux étapes (b) et (c) est de préférence de l'ordre de 7 à 8,5 , tout particulièrement de 7 à 8.

Différents modes de réalisation de l'opération de formation de bouillie selon l'invention sont décrits dans EP-A-520 862, FR-A-2 710 629, EP-A-670 813, EP-A- 670 814, WO 98/5409.

La bouillie de silice ainsi formée est ensuite séparée par filtration/lavage.

Cette étape peut être réalisée selon toute méthode convenable, par exemple à l'aide d'un filtre presse, d'un filtre à bande, d'un filtre rotatif sous vide ... D'une manière préférentielle, ladite opération de filtration/lavage est réalisée à l'aide d'un filtre rotatif sous vide.

Le lavage est réalisé jusqu'à ce que le taux d'anion résiduel, exprimé en sulfate de sodium, présent dans le gâteau de filtration obtenu soit inférieur à 5% en poids, de préférence inférieur à 3% en poids, exprimé par rapport au produit final.

Le gâteau de filtration est alors fluidifié.

L'étape de fluidification peut être réalisée par action mécanique par passage du gâteau dans un broyeur par exemple de type colloïdal ou à billes, ou par action mécanique dans un déliteur équipé de systèmes raclants, à pâles ...

Cette opération peut aussi être réalisée par addition d'eau ou d'une solution aqueuse d'un acide, acide sulfurique notamment, afin d'obtenir un gâteau de silice présentant une perte au feu supérieure à 80%, de préférence d'au moins 82%.

Si nécessaire, le pH du gâteau peut être descendu à 3 par ajout d'acide à cette étape.

Il y aura lieu, à cette étape (ainsi qu'à l'étape de filtration/lavage) de tenir compte du taux limite d'anion résiduel à ne pas dépasser. Cette étape de fluidification doit être réalisée en l'absence de sels d'aluminium.

Le gâteau de silice est ensuite séché par un moyen de séchage rapide, tout particulièrement par atomisation.

Le séchage par atomisation de la silice peut être réalisé d'une manière connue à l'aide de divers types d'atomiseur. L'homme de métier sait adapter le type d'atomiseur en fonction du type d'objets recherché (poudres ou billes). Des atomiseurs à turbine ou à buses permettent d'obtenir des poudres de diamètre médian supérieur à 20µm, de préférence supérieur à 25µm, pouvant aller jusqu'à 250µm ou des billes de diamètre médian pouvant aller jusqu'à 600µm.

Les poudres ou les billes obtenues ne sont pas broyées.

La silice selon l'invention, qu'elle se présente sous forme de poudre, ou de billes (de préférence sous forme de poudre), présente la propriété de se désagglomérer et/ou se disperser en éléments de taille inférieure (à celle de départ), au sein des compositions dentifrices, lors de la préparation desdites compositions, et permet ainsi d'épaissir lesdites compositions ou d'apporter de la texture à celles-ci.

Elle est aisément désagglomérable et dispersable en éléments de diamètre médian inférieur à 50µm, généralement inférieur à 20µm, tout particulièrement inférieur à 15µm, par cisaillement approprié dans la pâte dentifrice en cours de préparation.

D'une manière préférentielle, ladite silice à l'état désaggloméré et dispersé dans une formulation dentifrice (gel ou opaque) ne présente pas plus de 20%, de préférence pas plus de 15% et tout particulièrement pas plus de 6% en poids, d'éléments de diamètre supérieur à 51 μm.

La mesure granulométrique de la silice à l'état désaggloméré et dispersé dans la composition dentifrice est déterminée par diffraction laser selon la norme NF X 11-666 à l'aide d'un granulomètre laser sans ultrasons (comme déjà décrit ci-dessus). Cette mesure est effectuée sur une dispersion aqueuse contenant 10% en poids de la formulation dentifrice ; avant la mesure, cette dispersion, mise dans des flacons, a été préalablement agitée pendant 15 minutes par disposition desdits flacons dans un agitateur vibrant.

Les dentifrices obtenus présentent un aspect lisse et ne donnent pas de sensation granuleuse en bouche.

Selon l'invention, ladite silice peut être utilisée comme agent épaississant ou texturant à raison de 0,1 à 20%, de préférence de 0,5 à 15%, tout particulièrement de 1 à 10% du poids de la composition dentifrice.

Ladite composition dentifrice peut comprendre en outre d'autres ingrédients habituels, en particulier des agents abrasifs minéraux, insolubles dans l'eau, éventuellement des agents épaississants autres, des humectants ...

Comme agents abrasifs, on peut mentionner en particulier les silices abrasives, le carbonate de calcium, l'alumine hydratée, la bentonite, le silicate d'aluminium, le silicate de zirconium, les métaphosphates et phosphates de sodium, de potassium, de calcium et de magnésium. La quantité totale de poudre(s) abrasive(s) peut constituer de l'ordre de 5 à 50% du poids de la composition dentaire.

Parmi les agents épaississants autres, on peut mentionner, la gomme xanthane, la gomme guar, les carraghénanes, les dérivés de la cellulose, les alginates, en quantité pouvant aller jusqu'à 5% du poids de ladite composition ...

Parmi les agents humectants on peut citer par exemple le glycérol, le sorbitol, les polyéthylène glycols, les polypropylène glycols, le xylitol, en quantité de l'ordre de 2 à 85%, de préférence de l'ordre de 3 à 55% du poids de composition dentifrice exprimé en sec.

Ces compositions peuvent en outre comporter des agents tensio-actifs, des agents détergents, des colorants, des antibactériens, des dérivés fluorés, des opacifiants, des arômes, des édulcorants, des agents antitartre, antiplaque, des agents de blanchiment, du bicarbonate de sodium, des antiseptiques, des enzymes, des extraits naturels (camomille, thym...)...

Un deuxième objet de l'invention consiste en un procédé pour épaissir les compositions dentifrices ou apporter de la texture aux compositions dentifrices, par incorporation auxdites compositions de la silice épaississante de haute structure, dense et hautement désagglomérable et/ou dispersible, dont les caractéristiques ont été développées ci-dessus.

Un dernier objet de l'invention consiste en les compositions dentifrices comprenant la silice épaississante de haute structure, hautement désagglomérable et/ou dispersible et de préférence dense, dont les caractéristiques ont été développées ci-dessus, à l'état désaggloméré et/ou dispersé sous la forme d'éléments de diamètre médian inférieur d'au plus 40μm, généralement inférieur à 20μm, tout particulièrement inférieur à 15μm. De préférence, la quantité d'éléments de diamètre supérieur à 51μm ne dépasse pas 20%, de préférence 15%, tout particulièrement 6% en poids. Elles peuvent comprendre de 0,1 à 20%, de préférence de 0,5 à 15%, tout particulièrement de 1 à 10% de ladite silice de haute structure, hautement dispersible et de préférence dense.

Les exemples suivants sont donnés à titre illustratif.

| Formulation dentifrice modèle (gel) | |
|---|---|
| | parties en poids |
| - CMC (Blanose 12M31 P commercialisé par HERCULES) | 0,8 |
| - Sorbitol (Neosorb 70/70 commercialisé par ROQUETTE FRERES) | 65,5 |
| - Saccharinate de sodium | 0,2 |
| - Benzoate de sodium | 0,1 |
| - Monofluorophosphate de sodium (MFP) | 0,76 |
| - H2O | 7,98 |
| - silice abrasive (Tixosil 63 commercialisé par RHODIA) | 10 |
| - silice de l'invention | 9 |
| - Colorant FDC blue dye Nr 1 (0.12% dans H2O) | 0,8 |
| - Arôme : spearmint (commercialisé par MANE) | 0,7 |
| - Agent moussant : Sipon LCS 98 * (30% dans eau) de SIDOBRE-SINNOVA | 4,16 |

(suite)

| Formulation dentifrice modèle (pâte) | |
|---|---|
| - Sorbitol (Neosorb 70/70 de ROQUETTE FRERES) | 45 |
| - Polyethylene glycol PEG 1500 | 5 |
| - Saccharinate de sodium | 0,2 |
| - Fluorure de sodium | 0,08 |
| - Monofluorophosphate de sodium | 0,72 |
| - eau | 24,2 |
| - silice abrasive (Tixosil 63 commercialisé par RHODIA) | 10 |
| - silice de l'invention | 7 |
| - Dioxyde de titane | 1 |
| - Arôme spearmint | 1 |
| - Agent moussant (30% dans eau) : Texapon Z 95 P de COGNIS | 5 |

Mesure de la viscosité d'une formulation dentifrice

La viscosité est déterminée sur tube de pâte de 25 mm de diamètre, à des périodes déterminées à 37°C après préparation de la pâte ou du gel.

Le matériel de mesure utilisé est un viscosimètre Brookfield RVT équipé d'un dispositif hélipath. La pige T - E est utilisée à 5 r.p.m. La mesure est effectuée en descente après 90 secondes.

**EXEMPLE 1**

Dans un réacteur en acier inoxydable muni d'un système d'agitation par hélices et d'un chauffage par double enveloppe, on introduit :

- 660 litres d'eau
- 11,8 Kg de $Na_2SO_4$ (électrolyte)
- 323 litres de silicate de sodium aqueux, présentant un rapport pondéral $SiO_2/Na_2O$ égal 3,45 et une densité à 20 °C égale à 1,230.

La concentration en $SiO_2$ dans le pied de cuve est alors de 77 g/l. Le mélange est alors porté à une température de 82 °C tout en le maintenant sous agitation. On y introduit alors 395 litres d'acide sulfurique dilué de densité à 20 °C égale à 1,050 jusqu'à obtenir dans le milieu réactionnel une valeur de pH (mesurée à sa température) égale à 7,5.

On introduit ensuite conjointement dans le milieu de réaction 77 litres de silicate de sodium aqueux du type décrit ci-avant et 106 litres d'acide sufurique, également du type décrit ci-avant, cette introduction simultanée d'acide et de silicate étant réalisée de manière telle que le pH du milieu de réaction, pendant la période d'introduction, soit constamment égal à 7,5 ± 0,1. Après introduction de la totalité du silicate, on continue à introduire de l'acide dilué à un débit de 310 l/h, et ceci pendant 5 minutes.

Cette introduction complémentaire d'acide amène alors le pH du milieu à une valeur égale à 5,0.

La durée totale de la réaction est fixée à 85 mn.

On obtient une bouillie réactionnelle qui est filtrée et lavée au moyen d'un filtre rotatif sous vide.

Le gâteau de filtration est ensuite fluidifié par action mécanique. On obtient un gâteau de silice pompable dont la perte au feu est de 86%, gâteau qui est ensuite séché au moyen d'un atomiseur à buses.

La silice obtenue présente les caractéristiques suivantes :

- surface spécifique BET = 159 m$^2$/g
- surface spécifique CTAB = 156 m$^2$/g
- prise d'huile DOP = 320 ml/100g,
- pH = 7,0
- Na2SO4 = 2,8 % en poids
- D50 (μm) = 123μm (mesure au SYMPATEC HELOS)
- granulométrie MALVERN avec ultra-sons :

  . d50 = 33μm

. % de particules >51 µm = 22

- densité non-tassée = 0,19
- densité tassée = 0,21
- facteur de désagglomération $F_D$ = 13,3 (mesure au SYMPATEC HELOS)

Le pouvoir épaississant apporté par addition de 9 parties en poids de silice épaississante dans la formulation dentifrice gel modèle donnée ci-dessus et évalué par mesure de la viscosité de la formulation dentifrice comme mentionné ci-dessus après 3 semaines à 37°C, est donné au tableau suivant et comparé à la viscosité apportée dans les mêmes conditions par une silice épaississante commerciale, la silice Tixosil 43 commercialisée par RHODIA présentant les caractéristiques suivantes

- surface spécifique BET = 276 $m^2$/g,
- surface spécifique CTAB = 198 $m^2$/g,
- prise d'huile DOP = 348 ml/100g,
- pH = 7,0
- Na2SO4 = 2,8 % en poids

Cette silice commerciale a été obtenue par mise en oeuvre de la quantité totale de silicate dans le pied de cuve, ledit pied de cuve contenant plus de 100g/l de silicate (exprimé en silice) et broyage.

| Silice épaississante | exemple 1 | Tixosil 43 |
|---|---|---|
| D50 (µm) SYMPATEC HELOS | 123 µm | 10 µm |
| densité non-tassée | 0,19 | 0,10 |
| densité tassée | 0,21 | 0,12 |
| $F_D$ | 13,3 | 3 |
| diamètre médian dans la formulation dentifrice gel(µm) (SYMPATEC HELOS) | 9,8 | 9,4 |
| % d'éléments de diamètre >51µm dans la formulation dentifrice gel (SYMPATEC HELOS) | 1 | 1,2 |
| Viscosité Brookfield (mPas.) de la formulation dentifrice gel | 1 100 000 | 500000 |

On constate donc qu'à prises d'huile DOP voisines, les silices de l'invention non broyées, denses et hautement dispersibles présentent un pouvoir épaississant très nettement supérieur à une silice épaississante broyée commerciale. Dans les deux cas, la pâte dentifrice (gel) obtenue est d'aspect lisse ; on ne constate pas de sensation granuleuse en bouche.

Disposer d'une silice dense hautement épaississante représente un avantage économique particulièrement important : notamment moins de poussiérage, économie de frais de transport (volume moindre) et quantité moindre de silice à mettre oeuvre pour atteindre le même niveau de viscosité de la composition dentifrice.

**EXEMPLE 2**

Dans un réacteur en acier inoxydable muni d'un système d'agitation par hélices et d'un chauffage par double enveloppe, on introduit :

- 15 litres de silicate de densité à 20°C égale à 1230 kg/m3 et de rapport pondéral $SiO_2$/$Na_2O$ égal à 3,5,
- 529 litres d'eau.

La concentration en SiO2 dans le pied de cuve est de 6,4 g/l.
Le mélange obtenu, maintenu sous agitation, est porté à 75 °C par chauffage double enveloppe. Quand cette température est atteinte on procède à la réaction de précipitation. On introduit avec un débit de 142 l/h de l'acide sulfurique dilué de densité à 20°C égale à 1050 kg/m3 jusqu'à obtenir dans le milieu réactionnel une valeur de pH égale à 8,7. On introduit ensuite conjointement dans le milieu réactionnel du silicate de sodium aqueux du type décrit ci-avant à un débit de 388 l/h et de l'acide sulfurique, également du type décrit ci-avant, à un débit régulé de façon à maintenir la valeur du pH du milieu réactionnel égale à 8,7. Après 55 minutes d'addition simultanée, on arrête l'introduction du silicate.

On maintient ensuite l'introduction de l'acide sulfurique dilué de densité à 20°C égale à 1,05 de façon à amener la valeur du pH à 4,3. On maintient alors la bouillie réactionnelle à ce pH pendant 5 minutes.

On obtient ainsi une bouillie réactionnelle qui est filtrée et lavée au moyen d'un filtre rotatif sous vide de telle sorte que l'on récupère finalement un gâteau de silice dont la perte au feu est de 85,4 %.

Ce gâteau est ensuite fluidifié par action mécanique. Pendant cette opération de délitage, de l'acide sulfurique est introduit de façon à obtenir un pH du gâteau délité de 3,1. Le gâteau fluidifié et acidifié est ensuite séché au moyen d'un atomiseur à buses de diamètre 1,3 mm.

Les caractéristiques de la silice obtenue sont les suivantes :

- surface spécifique B.E.T. = 205 $m^2$/g
- surface spécifique C.T.A.B. = 165 $m^2$/g
- prise d'huile D.O.P. = 311 ml/100g
- pH = 4,0
- $Na_2SO_4$ = 0,9 %
- densité non tassée = 0,19
- densité tassée = 0,21
- granulométrie Malvem sans ultra-sons
  d50 = 180 μm
- granulométrie Malvem avec ultra-sons
  d50 = 27 μm
  % > 51 μm = 12
- facteur de désagglomération FD = 16,8 (SYMPATEC)

Les viscosités Brookfield obtenues sur les formules gel et pâte opaque décrites ci-avant sont reportées dans le tableau suivant :

|  | Exemple 2 | Tixosil 43 |
|---|---|---|
| 1. Formule gel |  |  |
| Viscosité Brookfield (mPas) après 1 semaine à 37°C | 780 000 | 420 000 |
| Granulométrie Sympatec |  |  |
| d50 (μm) | 8,3 | 8,4 |
| Aspect du gel | Lisse | lisse |
| Sensation en bouche | Non granuleux | Non granuleux |
| 2. Formule pâte opaque |  |  |
| Viscosité Brookfield (mPas) après 4 semaines à 37°C | 500 000 | 310 000 |
| Granulométrie Malvem d50 (μm)<br>% particules > 51 μm | 8,9<br>3,9 | 7,8<br>0,4 |
| Aspect de la pâte | Lisse | lisse |
| Sensation en bouche | Non granuleux | Non granuleux |

**EXEMPLE 3**

Dans un réacteur en acier inoxydable muni d'un système d'agitation par hélices et d'un chauffage par double enveloppe, on introduit :

- 15 litres de silicate de densité à 20°C égale à 1230 kg/m3 et de rapport pondéral SiO2 sur Na2O égal à 3,5,
- 529 litres d'eau.

La concentration en SiO2 dans le pied de cuve est de 6,4 g/l.

Le mélange obtenu, maintenu sous agitation, est porté à 75 °C par chauffage double enveloppe. Quand cette température est atteinte on procède à la réaction de précipitation. On introduit avec un débit de 142 l/h de l'acide sulfurique dilué de densité à 20°C égale à 1050 kg/m3 jusqu'à obtenir dans le milieu réactionnel une valeur de pH égale à 8,7. On introduit ensuite conjointement dans le milieu réactionnel du silicate de sodium aqueux du type décrit ci-avant à un débit de 388 l/h et de l'acide sulfurique, également du type décrit ci-avant, à un débit régulé de façon à maintenir la valeur du pH du milieu réactionnel égale à 8,7. Après 55 minutes d'addition simultanée, on arrête l'introduction du silicate.

On maintient ensuite l'introduction de l'acide sulfurique dilué de densité à 20°C égale à 1,05 de façon à amener la valeur du pH à 3,9. On maintient alors la bouillie réactionnelle à ce pH pendant 5 minutes.

On obtient ainsi une bouillie réactionnelle qui est filtrée et lavée au moyen d'un filtre rotatif sous vide de telle sorte que l'on récupère finalement un gâteau de silice dont la perte au feu est de 85,4 %.

Ce gâteau est ensuite fluidifié par action mécanique. Pendant cette opération de délitage, de l'acide sulfurique est introduit de façon à obtenir un pH du gâteau délité de 3,8. Le gâteau fluidifié et acidifié est ensuite séché au moyen d'un atomiseur turbine tournant à 9000 t/mn.

Les caractéristiques de la silice obtenue sont les suivantes :

- surface spécifique B.E.T. = 205 m$^2$/g
- surface spécifique C.T.A.B. = 164 m$^2$/g
- prise d'huile D.O.P. = 316 ml/100g
- pH = 6,0
- Na2SO4 = 1,4 %
- densité non tassée = 0,16
- densité tassée = 0,23
- granulométrie Malvern sans ultra-sons
     d50 = 34,4 µm
- granulomètre Malvern avec ultra-sons

     . d50 = 23,8 µm
     . % > 51 µm = 5

- facteur de désagglomération FD = 14,5 (SYMPATEC)

Les viscosités Brookfield obtenues sur les formules gel et pâte opaque décrites ci-avant sont reportées dans le tableau suivant :

|  | Exemple 3 | Tixosil 43 |
|---|---|---|
| 1. Formule gel |  |  |
| Viscosité Brookfield (mPas) après 1 semaine à 37°C | 760 000 | 420 000 |
| Granulométrie Sympatec d50 (µm) %>51 µm | 6,9 0 | 8,4 0 |
| Aspect du gel | Lisse | lisse |
| Sensation en bouche | Non granuleux | Non granuleux |
| 2. Formule pâte opaque |  |  |
| Viscosité Brookfield (mPas) après 4 semaines à 37°C | 500 000 | 310000 |
| Granulométrie Malvern d50 (µm) % particules > 51 µm | 8,3 3,3 | 7,8 0,4 |
| Aspect de la pâte | Lisse | lisse |
| Sensation en bouche | Non granuleux | Non granuleux |

**Revendications**

1. Utilisation, en tant qu'agent épaississant ou texturant dans les compositions dentifrices, d'une silice de précipitation présentant

- un pH de 3,5 à 9 , de préférence de 4 à 9, tout particulièrement de 5 à 8
- une prise d'huile DOP supérieure à 200ml/g, de préférence supérieure à 230ml/g, tout particulièrement supérieure à 250ml/g
- une surface spécifique CTAB de 70 à 250 m$^2$/g, de préférence de 100 à 200 m$^2$/g
- un diamètre médian, déterminé par diffraction laser sans ultra-sons, de 20µm à 600µm, de préférence de 25µm à 600µm
- un diamètre médian après désagglomération aux ultra-sons d'au plus 40µm, de préférence d'au plus 35µm
- un taux d'anion résiduel, exprimé en sulfate de sodium, inférieur à 5% en poids, de préférence inférieur à 3 % en poids.

2. Utilisation selon la revendication 1), **caractérisée en ce que** ladite silice présente, après désagglomération aux ultra-sons, un pourcentage en poids de particules de diamètre supérieur à 51µm d'au plus 30, de préférence d'au plus 25.

3. Utilisation selon la revendication 1) ou 2), **caractérisée en ce que** ladite silice présente une densité de remplissage à l'état tassé d'au moins 0,17g/ml, de préférence d'au moins 0,18 g/ml, tout particulièrement d'au moins 0,19 g/ml.

4. Utilisation selon la revendication 3), **caractérisée en ce que** ladite silice présente une densité de remplissage à l'état tassé d'au moins 0,20 g/ml.

5. Utilisation selon l'une quelconque des revendications 1) à 4), **caractérisée en ce que** ladite silice présente un facteur de désagglomération aux ultra-sons F$_D$ d'au moins 8, de préférence d'au moins 9,5.

6. Utilisation selon l'une quelconque des revendications 1) à 5), **caractérisée en ce que** ladite silice est sous forme de poudre de diamètre allant jusqu'à 250µm ou de billes de diamètre allant jusqu'à 600µm.

7. Utilisation selon l'une quelconque des revendications 1) à 6), **caractérisée en ce que** ladite silice est mise en oeuvre à raison de 0,1 à 20%, de préférence de 0,5 à 15%, tout particulièrement de 1 à 10% en poids dans lesdites compositions dentifrices.

8. Utilisation selon l'une quelconque des revendications 1) à 7), **caractérisée en ce que** ladite silice est, après incorporation dans lesdites compositions dentifrices, à l'état désaggloméré et/ou dispersé sous forme d'éléments de diamètre médian inférieur à 50µm, de préférence inférieur à 20µm, tout particulièrement inférieur à 15µm

9. Utilisation selon la revendication 8), **caractérisée en ce que** ladite silice à l'état désaggloméré et/ou dispersé ne présente pas plus de 20%, de préférence pas plus de 15%, tout particulièrement pas plus de 6% en poids d'éléments de diamètre supérieur à 51 µm.

10. Compositions dentifrices épaissies ou texturées obtenues par :

- incorporation d'une silice épaississante de haute structure, hautement désagglomérable et/ou dispersible présentant

  - un pH de 3,5 à 9 , de préférence de 4 à 9, tout particulièrement de 5 à 8
  - une prise d'huile DOP supérieure à 200ml/g, de préférence supérieure à 230ml/g, tout particulièrement supérieure à 250ml/g
  - une surface spécifique CTAB de 70 à 250 m$^2$/g, de préférence de 100 à 200 m$^2$/g
  - un diamètre médian, déterminé par diffraction laser sans ultra-sons, de 20µm à 600µm, de préférence de 25µm à 600µm
  - un diamètre médian après désagglomération aux ultra-sons d'au plus 40µm, de préférence d'au plus 35µm
  - un taux d'anion résiduel, exprimé en sulfate de sodium, inférieur à 5% en poids, de préférence inférieur à 3 % en poids ;

- désagglomération et/ou dispersion de ladite silice sous forme d'éléments de diamètre médian inférieur à 50µm, de préférence inférieur à 20µm, tout particulièrement inférieur à 15µm par cisaillement dans la pâte dentifrice en cours de préparation.

11. Compositions selon la revendication 10), **caractérisées en ce que** ladite silice à l'état désaggloméré et/ou dispersé ne présente pas plus de 20%, de préférence pas plus de 15%, tout particulièrement pas plus de 6% en poids d'éléments de diamètre supérieur à 51µm.

12. Compositions selon la revendication 10) ou 11), **caractérisées en ce que** ladite silice silice épaississante de haute structure, hautement désagglomérable et/ou dispersible présente, après désagglomération aux ultra-sons, un pourcentage en poids de particules de diamètre supérieur à 51µm d'au plus 30, de préférence d'au plus 25.

13. Compositions selon l'une quelconque des revendications 10) à 12), **caractérisées en ce que** ladite silice épaississante de haute structure, hautement désagglomérable et/ou dispersible présente une densité de remplissage à l'état tassé d'au moins 0,17g/ml, de préférence d'au moins 0,18 g/ml, tout particulièrement d'au moins 0,19 g/ml.

14. Compositions selon la revendication 13), **caractérisées en ce que** ladite silice épaississante de haute structure, hautement désagglomérable et/ou dispersible présente une densité de remplissage à l'état tassé d'au moins 0,20 g/ml.

15. Compositions selon l'une quelconque des revendications 10) à 14), **caractérisées en ce que** ladite silice épaississante de haute structure, hautement désagglomémble et/ou dispersible présente un facteur de désagglomération aux ultra-sons $F_D$ d'au moins 8, de préférence d'au moins 9,5.

16. Compositions selon l'une quelconque des revendications 10) à 15), **caractérisées en ce que** ladite silice épaississante de haute structure, hautement désagglomérable et/ou dispersible est sous forme de poudre de diamètre allant jusqu'à 250µm ou de billes de diamètre allant jusqu'à 600µm.

17. Compositions dentifrices selon l'une quelconque des revendications 10) à 16), obtenues par incorporation de 0,1 à 20%, de préférence de 0,5 à 15%, tout particulièrement de 1 à 10% de leur poids de ladite silice.


**Patentansprüche**

1. Verwendung als Verdickungsmittel oder Texturmittel in Zahnpflegemittelzusammensetzungen von Fällungskieselsäure, welche aufweist

   - einen pH von 3,5 bis 9, vorzugsweise von 4 bis 9, insbesondere von 5 bis 8

   - eine Ölzahl DOP größer als 200 ml/g, vorzugsweise größer als 230 ml/g, insbesondere größer als 250 ml/g

   - eine spezifische Oberfläche CTAB von 70 bis 250 m²/g, vorzugsweise von 100 bis 200 m²/g

   - einen mittleren Durchmesser, bestimmt durch Laserdiffraktion ohne Ultraschall, von 20 µm bis 600 µm, vorzugsweise von 25 µm bis 600 µm

   - einen mittleren Durchmesser nach Deagglomeration mit Ultraschall von höchstens 40 µm, vorzugsweise höchstens 35 µm

   - einen Anionen-Restgehalt, ausgedrückt in Natriumsulfat, von weniger als 5 Gew.-%, vorzugsweise weniger als 3 Gew.-%.

2. Verwendung gemäß Anspruch 1, **dadurch gekennzeichnet, dass** die Kieselsäure nach Deagglomeration mit Ultraschall einen Gewichtsprozentsatz an Partikeln mit einem Durchmesser größer als 51 µm von höchstens 30, vorzugsweise höchstens 25 aufweist.

3. Verwendung gemäß Anspruch 1 oder 2, **dadurch gekennzeichnet, dass** die Kieselsäure in komprimiertem Zustand eine Fülldichte von wenigstens 0,17 g/ml, vorzugsweise wenigstens 0,18 g/ml, insbesondere wenigstens 0,19 g/ml aufweist.

4. Verwendung gemäß Anspruch 3, **dadurch gekennzeichnet, dass** die Kieselsäure in komprimiertem Zustand eine Fülldichte von wenigstens 0,20 g/ml aufweist.

**5.** Verwendung gemäß einem der Ansprüche 1 bis 4, **dadurch gekennzeichnet, dass** die Kieselsäure einen Deagglomerationsfaktor bei Ultraschall $F_D$ von wenigstens 8, vorzugsweise wenigstens 9,5 aufweist.

**6.** Verwendung gemäß einem der Ansprüche 1 bis 5, **dadurch gekennzeichnet, dass** die Kieselsäure in Pulverform mit einem Durchmesser von bis zu 250 µm, oder in Kugelform mit einem Durchmesser bis 600 µm vorliegt.

**7.** Verwendung gemäß einem der Ansprüche 1 bis 6, **dadurch gekennzeichnet, dass** die Kieselsäure zu 0,1 bis 20 %, vorzugsweise 0,5 bis 15 %, insbesondere 1 bis 10 Gew.-%, in den Zahnpflegemittelzusammensetzungen verwendet wird.

**8.** Verwendung gemäß einem der Ansprüche 1 bis 7, **dadurch gekennzeichnet, dass** die Kieselsäure nach Einschluss in die Zahnpflegemittelzusammensetzungen in deagglomeriertem und/oder dispergiertem Zustand in Form von Teilen mit einem mittleren Durchmesser von weniger als 50 µm, vorzugsweise weniger als 20 µm, insbesondere weniger als 15 µm vorliegt.

**9.** Verwendung gemäß Anspruch 8, **dadurch gekennzeichnet, dass** die Kieselsäure in deagglomeriertem und/oder dispergiertem Zustand nicht mehr als 20 %, vorzugsweise nicht mehr als 15 %, insbesondere nicht mehr als 6 Gew.-% der Teile mit einem Durchmesser von mehr als 51 µm darstellt.

**10.** Verdickte oder texturierte Zahnpflegemittelzusammensetzungen, die erhalten wurden durch:

- Beimischen einer verdickenden Kieselsäure mit hoher Struktur, welche in hohem Maße deagglomerierbar und/oder dispergierbar ist und aufweist

  • einen pH von 3,5 bis 9, vorzugsweise von 4 bis 9, insbesondere von 5 bis 8

  • eine Ölzahl DOP größer als 200 ml/g, vorzugsweise größer als 230 ml/g, insbesondere größer als 250 ml/g

  • eine spezifische Oberfläche CTAB von 70 bis 250 m$^2$/g, vorzugsweise von 100 bis 200 m$^2$/g

  • einen mittleren Durchmesser, bestimmt durch Laserdiffraktion ohne Ultraschall, von 20 µm bis 600 µm, vorzugsweise von 25 µm bis 600 µm

  • einen mittleren Durchmesser nach Deagglomeration mit Ultraschall von höchstens 40 µm, vorzugsweise höchstens 35 µm

  • einen Anionen-Restgehalt, ausgedrückt in Natriumsulfat, von weniger als 5 Gew.-%, vorzugsweise weniger als 3 Gew.-%.;

- Deagglomeration und/oder Dispersion der Kieselsäure in Form von Teilen mit einem mittleren Durchmesser kleiner als 50 µm, vorzugsweise kleiner als 20 µm, insbesondere kleiner als 15 µm, durch Abscheren in den Zahnpflegemittelteig im Laufe der Herstellung.

**11.** Zusammensetzungen gemäß Anspruch 10, **dadurch gekennzeichnet, dass** die Kieselsäure in deagglomeriertem und/oder dispergiertem Zustand nicht mehr als 20 %, vorzugsweise nicht mehr als 15 %, insbesondere nicht mehr als 6 Gew.-% an Teilen mit einem Durchmesser von mehr als 51 µm aufweist.

**12.** Zusammensetzungen gemäß Anspruch 10 oder 11, **dadurch gekennzeichnet, dass** die Kieselsäure, verdickende Kieselsäure mit hoher Struktur, welche in hohem Maße deagglomerierbar und/oder dispergierbar ist, nach Deagglomeration mit Ultraschall einen Gewichtsprozentsatz an Partikeln mit einem Durchmesser größer als 51 µm von höchstens 30, vorzugsweise höchstens 25 aufweist.

**13.** Zusammensetzungen gemäß einem der Ansprüche 10 bis 12, **dadurch gekennzeichnet, dass** die verdickende Kieselsäure mit hoher Struktur, welche in hohem Maße deagglomerierbar und/oder dispergierbar ist, in komprimiertem Zustand eine Fülldichte von wenigstens 0,17 g/ml, vorzugsweise wenigstens 0,18 g/ml, insbesondere wenigstens 0,19 g/ml, aufweist.

**14.** Zusammensetzungen gemäß Anspruch 13, **dadurch gekennzeichnet, dass** die verdickende Kieselsäure mit hoher Struktur, welche in hohem Maße deagglomerierbar und/oder dispergierbar ist, in komprimiertem Zustand eine Fülldichte von wenigstens 0,20 g/ml aufweist.

**15.** Zusammensetzungen gemäß einem der Ansprüche 10 bis 14, **dadurch gekennzeichnet, dass** die verdickende Kieselsäure mit hoher Struktur, welche in hohem Maße deagglomerierbar und/oder dispergierbar ist, einen Deagglomerationsfaktor bei Ultraschall $F_D$ von wenigstens 8, vorzugsweise wenigstens 9,5 aufweist.

**16.** Zusammensetzungen gemäß einem der Ansprüche 10 bis 15, **dadurch gekennzeichnet, dass** die verdickende Kieselsäure mit hoher Struktur, welche in hohem Maße deagglomerierbar und/oder dispergierbar ist, in Pulverform mit einem Durchmesser bis zu 250 µm oder in Kugelform mit einem Durchmesser bis zu 600 µm vorliegt.

**17.** Zahnpflegezusammensetzungen gemäß einem der Ansprüche 10 bis 16, welche durch Beimischen von Kieselsäure zu 0,1 bis 20 %, vorzugsweise 0,5 bis 15 %, insbesondere 1 bis 10 % ihres Gewichts erhalten werden.

**Claims**

**1.** Use, as a thickener or texturing agent in toothpaste compositions, of a precipitated silica having

- a pH from 3.5 to 9, preferably from 4 to 9 and most particularly from 5 to 8
- a DOP oil uptake of greater than 200 ml/g, preferably greater than 230 ml/g and most particularly greater than 250 ml/g
- a CTAB specific surface area from 70 to 250 $m^2$/g and preferably from 100 to 200 $m^2$/g
- a median diameter determined by laser diffraction without ultrasound from 20 µm to 600 µm, preferably from 25 µm to 600 µm
- a median diameter after ultrasound-mediated disintegration of not more than 40 µm and preferably of not more than 35 µm
- a residual anion content, expressed as sodium sulfate, of less than 5% by weight and preferably less than 3% by weight.

**2.** Use according to Claim 1, **characterized in that** said silica has, after ultrasound-mediated disintegration, a weight percentage of particles greater than 51 µm in diameter of not more than 30 and preferably not more than 25.

**3.** Use according to Claim 1 or 2, **characterized in that** said silica has a tamped filling density of at least 0.17 g/ml, preferably of at least 0.18 g/ml and most particularly of at least 0.19 g/ml.

**4.** Use according to Claim 3, **characterized in that** said silica has a tamped filling density of at least 0.20 g/ml.

**5.** Use according to any one of Claims 1 to 4, **characterized in that** said silica has an ultrasound-mediated disintegration factor $F_D$ of at least 8 and preferably of at least 9.5.

**6.** Use according to any one of Claims 1 to 5, **characterized in that** said silica is in the form of a powder with a diameter ranging up to 250 µm or of beads with a diameter ranging up to 600 µm.

**7.** Use according to any one of Claims 1 to 6, **characterized in that** said silica is used in a proportion of from 0.1% to 20%, preferably from 0.5% to 15% and most particularly from 1% to 10% by weight in said toothpaste compositions.

**8.** Use according to any one of Claims 1 to 7, **characterized in that** said silica is, after incorporation in said toothpaste compositions, in disintegrated and/or dispersed form, in the form of elements with a median diameter of less than 50 µm, preferably less than 20 µm and most particularly less than 15 µm.

**9.** Use according to Claim 8, **characterized in that** said silica in disintegrated and/or dispersed form does not contain more than 20%, preferably not more than 15% and most particularly not more than 6% by weight, of elements greater than 51 µm in diameter.

**10.** Thickened or textured toothpaste compositions obtained by:

- incorporating a highly structured, highly disintegrable and/or dispersible thickening silica having

  . a pH from 3.5 to 9, preferably from 4 to 9 and most particularly from 5 to 8
  . a DOP oil uptake of greater than 200 ml/g, preferably greater than 230 ml/g and most particularly greater than 250 ml/g
  . a CTAB specific surface area from 70 to 250 $m^2$/g and preferably from 100 to 200 $m^2$/g
  . a median diameter determined by laser diffraction without ultrasound from 20 $\mu$m to 600 $\mu$m, preferably from 25 $\mu$m to 600 $\mu$m
  . a median diameter after ultrasound-mediated disintegration of not more than 40 $\mu$m and preferably of not more than 35 $\mu$m
  . a residual anion content, expressed as sodium sulfate, of less than 5% by weight and preferably less than 3% by weight;

- disintegrating and/or dispersing said silica in the form of elements with a median diameter of less than 50 $\mu$m, preferably less than 20 $\mu$m and most particularly less than 15 $\mu$m, by shearing in the toothpaste during preparation.

11. Compositions according to Claim 10, **characterized in that** said silica in disintegrated and/or dispersed form does not contain more than 20%, preferably not more than 15% and most particularly not more than 6% by weight, of elements greater than 51 $\mu$m in diameter.

12. Compositions according to Claim 10 or 11, **characterized in that** said silica, a highly structured, highly disintegrable and/or dispersible thickening silica, has, after ultrasound-mediated disintegration, a weight percentage of particles greater than 51 $\mu$m in diameter of not more than 30 and preferably not more than 25.

13. Compositions according to any one of Claims 10 to 12, **characterized in that** said highly structured, highly disintegrable and/or dispersible thickening silica has a tamped filling density of at least 0.17 g/ml, preferably of at least 0.18 g/ml and most particularly of at least 0.19 g/ml.

14. Compositions according to Claim 13, **characterized in that** said highly structured, highly disintegrable and/or dispersible thickening silica has a tamped filling density of at least 0.20 g/ml.

15. Compositions according to any one of Claims 10 to 14, **characterized in that** said highly structured, highly disintegrable and/or dispersible thickening silica has an ultrasound-mediated disintegration factor $F_D$ of at least 8 and preferably of at least 9.5.

16. Compositions according to any one of Claims 10 to 15, **characterized in that** said highly structured, highly disintegrable and/or dispersible thickening silica is in the form of a powder with a diameter ranging up to 250 $\mu$m or of beads with a diameter ranging up to 600 $\mu$m.

17. Toothpaste compositions according to any one of Claims 10 to 16, which are obtained by incorporation of from 0.1% to 20%, preferably from 0.5% to 15% and most particularly from 1% to 10% of their weight, of said silica.